# EUROPEAN PATENT SPECIFICATION

(11) **EP 1 658 050 B1**
(45) Date of publication and mention of the grant of the patent: **25.06.2008**
(21) Application number: 04769442.7
(22) Date of filing: 27.08.2004
(51) Int. Cl.: A61K 9/107, A61K 31/337, A61K 38/13

(54) **SELF-NANOEMULSIFYING OILY FORMULATION FOR THE ADMINISTRATION OF POORLY WATER-SOLUBLE DRUGS**
SELBST-NANOEMULGIERENDE ÖLIGE FORMULIERUNG ZUR VERABREICHUNG VON SCHWER WASSERLÖSLICHEN ARZNEIMITTELN
FORME GALENIQUE HUILEUSE AUTO-NANOEMULSIFIANTE POUR L'ADMINISTRATION DE MEDICAMENTS DIFFICILEMENT HYDROSOLUBLES

(30) Priority: 29.08.2003 EP 03292141; 29.08.2003 US 498663 P
(43) Date of publication of application: 24.05.2006
(73) Proprietor: Novagali Pharma S.A., 91000 Evry (FR); YISSUM RESEARCH DEVELOPMENT COMPANY OF THE HEBREW UNIVERSITY OF JERUSALEM, Givat Ram, Jerusalem 91390 (IL)
(72) Inventor: GARRIGUE, Jean, Sébastien, 91370 Verrieres le Buisson (FR); LAMBERT, Gregory, 92200 Chatenay Malabri (FR); RAZAFINDRATSITA, Alain, 92160 Antony (FR); BENITA, Simon, 90805 Mevasseret Zion (IL); YANG, Shicheng Dept.Industrial&Physical Pharmacy, IN 47907-1336 (US); GURSOY, Neslihan, Jerusalem (IL)
(74) Representative: de Mareüil-Villette, Caroline
(86) International application number: PCT/IB2004/003077
(87) International publication number: WO 2005/020962

(56) References cited:
- EP-A- 1 340 497
- WO-A-00/78247
- WO-A-02/43765
- WO-A-95/11039
- WO-A-99/45918
- US-A1- 2002 156 124
- US-B1- 6 458 373
- US-B1- 6 509 370

## Description

The present invention relates to a pharmaceutical excipient formulation, more particularly to a pharmaceutical excipient composition consisting in a self-nanoemulsifying oily formulation (SNEOF) enhancing the absorption of poorly water soluble drugs, particularly the oral absorption of taxoids and cytotoxic agents, based on improved dissolution and absorption of the drug; and providing a dose-AUC linear pharmacokinetic of the drug.

The clinical use of some drugs is only possible if a specific drug delivery system is developed to transport them to their therapeutic target in the human body. This problem is particularly critical for water insoluble or poorly water soluble compounds for which direct injections may be impossible or problematic.

A few examples of therapeutic substances, which are poorly hydrosoluble, are the following: Palmitoyl Rhizoxin, Penclomedine, Vitamin A and its derivatives (retinoic acid, isotretinoin, etc.), Tamoxifen, Etoposide, Campothecin, Navelbine, Valproic acid, Tacrolimus, Sirolimus (Rapamycin), Cyclosporin A, Clarithromicin, Testosterone, Estradiol, Progesterone, Ciprofloxacine, Fenofibrate, Benzafibrate, Azithromicine, Itraconazole, Miconazole, Propofol, Brimonidine, Latanoprost, and Paclitaxel.

Paclitaxel, one of the best known taxoids, disrupts tubulin dynamics. It has a significant clinical activity against a broad range of tumor types including breast, lung, head and neck, bladder, and platinum-refractory ovarian carcinoma (E. K. Rowinsky. The development and clinical utility of the taxoid class of antimicrotubule chemotherapy agents. Annu Rev Med. 48: 353-74 (1997)). However, paclitaxel has a low therapeutic index. It is a complex diterpenoid product, with a bulky, extended fused ring system as well as a number of hydrophobic substituents, which lead to its poor solubility in water (1 µg/ml) resulting in serious formulation problems (R. T. Liggins, W. L. Hunter, H. M. Burt. Solid-state characterization of paclitaxel. J Pharm Sci. 86: 1458-63 (1997)). It is highly lyophobic and the solubility of paclitaxel in lipophilic solvents, such as soybean oil is quite low and precludes the use of simple oil-in-water emulsions for formulation considerations. The commercially available product, Taxol®, is currently formulated for systemic administration in a mixture of ethanol and polyoxyethylated castor oil (Cremophor EL); the latter appears to be primarily responsible for drug related hypersensitivity reactions, rather than the drug itself (R. E. Gregory, A. F. De Lisa. Paclitaxel: a new antineoplastic agent for refractory ovarian cancer. Clin Pharm. 12: 401-15 (1993)). Moreover, polyoxyethylated castor oil also causes the nonlinear pharmacokinetic behavior of paclitaxel (A. Sparreboom, O. van Tellingen, W. J. Nooijen, J.H. Beijnen. Nonlinear pharmacokinetics of paclitaxel in mice results from the pharmaceutical vehicle Cremophor EL. Cancer Res. 56: 2112-5 (1996); O. van Tellingen, M. T. Huizing, V. R. Panday, J. H. Schellens, W. J. Nooijen, J. H. Beijnen. Cremophor EL causes (pseudo-) non-linear pharmacokinetics of paclitaxel in patients. Br J Cancer 81: 330-5 (1999)).

The current approaches for reducing the side effects of the actual commercial product are mainly focused on developing formulations that are devoid of polyoxyethylated castor oil. Several attempts have been made to deliver paclitaxel using alternative systems, such as nanoparticles (R. Cavalli, O. Caputo, M. R. Gasco. Preparation and characterization of solid lipid nanospheres containing paclitaxel. Eur J Pharm Sci. 10: 305-9 (2000); S. S. Feng, G. F. Huang, L. Mu. Nanospheres of biodegradable polymers: a system for clinical administration of an anticancer drug paclitaxel (Taxol). [In Process Citation]. Ann Acad Med Singapore. 29: 633-9 (2000)), liposomes (P. Crosasso, M. Ceruti, P. Brusa, S. Arpicco, F. Dosio, L. Cattel. Preparation, characterization and properties of sterically stabilized paclitaxel-containing liposomes. J Controlled Release. 63: 19-30 (2000); A. Sharma, R. M. Straubinger. Novel taxol formulations: preparation and characterization of taxol-containing liposomes. Pharm Res. 11: 889-96 (1994)), water-soluble prodrugs (J. M. Terwogt, B. Nuijen, W. W. T. B. Huinink, J. H. Beijnen. Alternative formulations of paclitaxel. Cancer Treat Rev. 23: 87-95 (1997); A. Pendri, C. D. Conover, R. B. Greenwald. Antitumor activity of paclitaxel-2'-glycinate conjugated to poly(ethylene glycol): a water-soluble prodrug. Anticancer Drug Des. 13: 387-95 (1998)), emulsions (P. P. Constantinides, K. J. Lambert, A. K. Tustian, B. Schneider, S. Lalji, W. Ma, B. Wentzel, D. Kessler, D. Worah, and S. C. Quay. Formulation development and antitumor activity of a filter-sterilizable emulsion of paclitaxel. Pharm Res. 17: 175-82 (2000); B. B. Lundberg. A submicron lipid emulsion coated with amphipathic polyethylene glycol for parenteral administration of paclitaxel (Taxol®). J Pharm Pharmacol. 49:16-21 (1997); P. Kan, Z. B. Chen, C. J. Lee, I. M. Chu. Development of nonionic surfactant/phospholipid o/w emulsion as a paclitaxel delivery system. J Controlled Release. 58: 271-8 (1999), P. Simamora, R. M. Dannenfelser, S. E. Tabibi, S. H. Yalkowsky. Emulsion formulations for intravenous administration of paclitaxel. PDA J Pharm Sci Technol. 52: 170-2 (1998)) and microspheres (R. T. Liggins, S. D'Amours, J. S. Demetrick, L. S. Machan, H. M. Burt. Paclitaxel loaded poly(L-lactic acid) microspheres for the prevention of intraperitoneal carcinomatosis after a surgical repair and tumor cell spill [In Process Citation]. Biomaterials. 21: 1959-69 (2000); Y. M. Wang, H. Sato, I. Adachi, I. Horikoshi. Preparation and characterization of poly(lactic-co-glycolic acid) microspheres for targeted delivery of a novel anticancer agent, taxol. Chem Pharm Bull (Tokyo). 44: 1935-40 (1996)). However, the success is for the moment still limited. None of these alternatives has reached the stage of replacing polyoxyethylated castor oil based vehicle in the clinical application.

Another approach to overcome the hypersensitivity reactions resulting from polyoxyethylated castor oil can be the design of oral formulations of paclitaxel (J. M. M. Terwogt, M. M. Malingre, J. H. Beijnen, W. W. B. Huinink, H. Rosing, F. J. Koopman, O. van Tellingen, M. Swart, and J. H. M. Schellens. Coadministration of oral cyclosporin A enables oral therapy with paclitaxel. Clin Cancer Res. 5: 3379-84 (1999)). Oral administration of paclitaxel would, thus, prevent the adverse effects caused by the vehicle substance polyoxyethylated castor oil and offer additional advantages over intravenous administration, including elimination of the need for frequent visits to the outpatient clinic and easier chronic administration (R. T. Dorr. Pharmacology and toxicology of Cremophor EL diluent. Ann Pharmacother. 28: S11-4 (1994); A. J. ten Tije, J. Verweij, W. J. Loos, and A. Sparreboom. Pharmacological effects of formulation vehicles : implications for cancer chemotherapy. Clin Pharmacokinet 42: 665-85 (2003)). However, preclinical studies have suggested that paclitaxel is not significantly absorbed after oral administration; the systemic bioavailability in humans after oral paclitaxel administration is less than 6% (J. M. M. Terwogt, M. M. Malingre, J. H. Beijnen, W. W. B. Huinink, H. Rosing, F. J. Koopman, O. van Tellingen, M. Swart, and J. H. M. Schellens. Coadministration of oral cyclosporin A enables oral therapy with paclitaxel. Clin Cancer Res. 5: 3379-84 (1999)). The explanations proposed to account for the poor oral bioavailability of paclitaxel are multifactorial. The most likely explanations are its affinity for the membrane-bound drug efflux pump P-glycoprotein (P-gp), metabolization by cytochromes P450 (2C8 and 3A4) and poor water solubility (R. T. Liggins, W. L. Hunter, H. M. Burt. Solid-state characterization of paclitaxel. J Pharm Sci. 86: 1458-63 (1997); J. van Asperen, O. van Tellingen, A. Sparreboom, A. H. Schinkel, P. Borst, W. J. Nooijen, and J. H. Beijnen. Enhanced oral bioavailability of paclitaxel in mice treated with the P-glycoprotein blocker SDZ PSC 833. Br J Cancer. 76: 1181-3 (1997); C. D. Britten, S. D. Baker, L. J. Denis, T. Johnson, R. Drengler, L. L. Siu, K. Duchin, J. Kuhn, and E. K. Rowinsky. Oral paclitaxel and concurrent cyclosporin A: targeting clinically relevant systemic exposure to paclitaxel. Clin Cancer Res. 6: 3459-68 (2000)). Moreover, the polyethoxylated castor oil (Cremophor EL) was shown to be in part responsible of the low bioavailability and poor pharmacokinetic linearity of orally administered Taxol® (H. A. Bardelmeijer, M. Ouwehand, M. M. Malingre, J. H. Schellens, J. H. Beijnen, and O. van Tellingen. Entrapment by Cremophor EL decreases the absorption of paclitaxel from the gut. Cancer Chemother Pharmacol 49: 119-125 (2002); M. M. Malingre, J. H. Schellens, O. Van Tellingen, M. Ouwehand, H. A. Bardelmeijer, H. Rosing, F. J. Koopman, M. E. Schot, W. W. Ten Bokkel Huinink, and J. H. Beijnen. The co-solvent Cremophor EL limits absorption of orally administered paclitaxel in cancer patients. Br J Cancer 85: 1472-1477 (2001)).

A number of studies have been carried out to verify in both animals and patients if the oral bioavailability of paclitaxel could be improved when the drug is administered with P-gp or cytochrome P450 inhibitors (R. T. Dorr. Pharmacology and toxicology of Cremophor EL diluent. Ann Pharmacother. 28: S11-4 (1994); J. van Asperen, O. van Tellingen, A. Sparreboom, A. H. Schinkel, P. Borst, W. J. Nooijen, and J. H. Beijnen. Enhanced oral bioavailability of paclitaxel in mice treated with the P-glycoprotein blocker SDZ PSC 833. Br J Cancer. 76: 1181-3 (1997); C. D. Britten, S. D. Baker, L. J. Denis, T. Johnson, R. Drengler, L. L. Siu, K. Duchin, J. Kuhn, and E. K. Rowinsky. Oral paclitaxel and concurrent cyclosporin A: targeting clinically relevant systemic exposure to paclitaxel. Clin Cancer Res. 6: 3459-68 (2000)). Cyclosporine A (CsA), a well-known immunosuppressive agent, was shown to be one of the most promising P-gp inhibitors to enhance the oral absorption of paclitaxel (J. M. M. Terwogt, M. M. Malingre, J. H. Beijnen, W. W. B. Huinink, H. Rosing, F. J. Koopman, O. van Tellingen, M. Swart, and J. H. M. Schellens. Coadministration of oral cyclosporin A enables oral therapy with paclitaxel. Clin Cancer Res. 5: 3379-84 (1999); C. D. Britten, S. D. Baker, L. J. Denis, T. Johnson, R. Drengler, L. L. Siu, K. Duchin, J. Kuhn, and E. K. Rowinsky. Oral paclitaxel and concurrent cyclosporin A: targeting clinically relevant systemic exposure to paclitaxel. Clin Cancer Res. 6: 3459-68 (2000)). CsA is a registered drug and thus is more readily available for clinical studies. The concomitant use of cyclosporin A for oral Taxol administration led to an increased AUC of paclitaxel (bioavailability of 20%). Nevertheless, this AUC enhancement was only reached for low doses. On the contrary, at higher doses administration showed a non linear pharmacokinetic in both rodents and human. A five fold increase (from 60 to 300mg/m²) of the dose in human only led to a 2 fold increase of plasmatic AUC (M. M. Malingre, J. M. Terwogt, J. H. Beijnen, H. Rosing, F. J. Koopman, O. van Tellingen, K. Duchin, W. W. Huinink, M. Swart, J. Lieverst, and J. H. Schellens. Phase I and pharmacokinetic study of oral paclitaxel. J Clin Oncol 18: 2468-2475. (2000)). This non linear dose-AUC relationship is a significant obstacle to the use of oral Taxol®. Moreover, oral Taxol^{®} exhibited a poor tolerability in patients and occasioned acute gastro-intestinal disorders such as nausea and vomiting. The formulation contains a high amount of ethanol (50%) and high clinically required doses lead to a significant amount of ingested ethanol. Moreover, the formulation is very bitter due to the presence of Cremophor EL. In conclusions, the oral administration of Taxol® is greatly limited by the bad tolerability after ingestion. The non linear pharmacokinetic lead the clinician to investigate high doses of Taxol® (> 300mg/m² paclitaxel) (M. M. Malingre, J. M. Terwogt, J. H. Beijnen, H. Rosing, F. J. Koopman, O. van Tellingen, K. Duchin, W. W. Huinink, M. Swart, J. Lieverst, and J. H. Schellens. Phase I and pharmacokinetic study of oral paclitaxel. J Clin Oncol 18: 2468-2475. (2000)). In a phase II trial of weekly oral paclitaxel plus cyclosporine in patients with advanced non-small-cell lung cancer, interpatient variability was calculated at 40 to 45% and intra-individual variability at 15% (C. M. Kruijtzer, J. H. Schellens, J. Mezger, M. E. Scheulen, U. Keilholz, J. H. Beijnen, H. Rosing, R. A. Mathot, S. Marcus, H. van Tinteren, and P. Baas. Phase II and pharmacologic study of weekly oral paclitaxel plus cyclosporine in patients with advanced non-small-cell lung cancer. J Clin Oncol 20: 4508-16 (2002)). Those points also represent important Taxol® limitations in the oral paclitaxel treatment.

Recently, it was reported that self-emulsifying oily formulation (SEOF) consisting of isotropic mixtures of oil and surfactants could significantly improve the oral availability of poorly absorbed, hydrophobic and/or lipophilic drugs (T. Gershanik, S. Benita. Self-dispersing lipid formulations for improving oral absorption of lipophilic drugs. Eur J Pharm Biopharm. 50: 179-88 (2000)). SEOFs are composed of natural or synthetic oils, surfactants and one or more hydrophilic solvents and co-solvents. The principal characteristic of SEOFs is their ability to form fine oil-in-water emulsions or microemulsions upon mild agitation following dilution by aqueous phases. These formulations can disperse in the gastrointestinal lumen to form microemulsions or fine emulsions, upon dilution with gastrointestinal fluids. In *in-vivo* absorption studies in non-fasting dogs, SEOFs elicited at least a three-fold greater Cₘₐₓ and AUC of a lipophilic naphthalene derivative than that of the drug in any other dosage form (N. H. Shah, M. T. Carvajal, C. I. Patel, M. H. Infeld, A. W. Malick. Self-emulsifying drug delivery systems (SEDDS) with polyglycolyzed glycerides for improving in vitro dissolution and oral absorption of lipophilic drugs. Int J Pharm. 106: 15-23 (1994)). The absorption of ontazolast in rats was significantly enhanced by all lipid-based formulations (D. J. Hauss, S. E. Fogal, J. V. Ficorilli, C. A. Price, T. Roy, A. A. Jayaraj, and J. J. Kierns. Lipid-based delivery systems for improving the bioavailability and lymphatic transport of a poorly water-soluble LTB4 inhibitor. J Pharm Sci. 87: 164-9 (1998)). Microemulsions have successfully been used to improve drug solubilization/dissolution and/or intestinal absorption of poorly absorbed drugs including CsA (P. P. Constantinides. Lipid microemulsions for improving drug dissolution and oral absorption: physical and biopharmaceutical aspects. Pharm Res. 12: 1561-72 (1995); S. Tenjarla. Microemulsions: an overview and pharmaceutical applications. Crit Rev Ther Drug Carrier Syst. 16: 461-521 (1999)).

Traditional surfactants are known to entrap lyophobic drugs. For example, addition of Cremophor EL to the formulation of oral drug preparations resulted in significantly diminished drug uptake and reduced circulating concentrations. The drawbacks presented by the presence of Cremophor EL or Tween 80 in drug formulations have instigated extensive research to develop alternative delivery forms. Currently, several strategies are in progress to develop Tween 80 and Cremophor EL-free formulations of docetaxel and paclitaxel, which are based on pharmaceutical, chemical or biological strategies (A. J. ten Tije, J. Verweij, W. J. Loos, and A. Sparreboom. Pharmacological effects of formulation vehicles: implications for cancer chemotherapy. Clin Pharmacokinet 42: 665-85 (2003); H. A. Bardelmeijer, M. Ouwehand, M. M. Malingre, J. H. Schellens, J. H. Beijnen, and O. van Tellingen. Entrapment by Cremophor EL decreases the absorption of paclitaxel from the gut. Cancer Chemother Pharmacol 49: 119-125 (2002)).

The rationale of a self-emulsifying oily formulation for the administration of oral paclitaxel lies in the better solubilization and absorption of paclitaxel and concomitant bioavailability variability reduction.

There is a continuing need for taxane compositions and formulations which provide a more efficient means of administering taxanes without causing undesired side effects and which have improved stability and longer shelf life.

An object of the instant invention is a pharmaceutical composition in a form of an anhydrous self-nanoemulsifying oily formulation comprising:
- one or more therapeutic agent(s) which have low solubility in water or are water-insoluble,
- vitamin E,
- one co-solvent selected from propylene glycol and ethanol and mixture thereof
- one surfactant selected from tyloxapol and mixture of tyloxapol and TPGS optionally,
- a bioenhancer.

In a specific aspect of the instant invention, the pH of the composition can be reduced to further improve the stability of the therapeutic agent. In some embodiments this is accomplished by the addition of an acidic pH adjuster which is selected from the group comprising ascorbic acid, citric acid, tartaric acid, lactic acid, oxalic acid, formic acid, benzene sulphonic acid, benzoic acid, maleic acid, glutamic acid, succinic acid, aspartic acid, diatrizoic acid, and acetic acid. The acidifying agent may also be an inorganic acid, including, but not limited to, hydrochloric acid, sulphuric acid, phosphoric acid, and nitric acid. An anhydrous organic acid, like anhydrous citric acid, may preferably be used in the composition.

In another specific embodiment of the pharmaceutical composition vitamin E is from 2 to 6% (w/w) of the final composition.

According to the invention, the one or more therapeutic agent(s) is selected from the group comprising anti-fungal drugs, anti-viral drugs, antibiotic drugs, anti-inflammatory drugs, anti-cancer drugs, analgesics, antidepressants, antipsychotics, hormones, antacids, coronary vasodilators, cerebral vasodilators, psychotropics, antineoplastics, stimulants, anti-histamines, vasodilators, anti-arrythmics, anti-hypertensive drugs, vasoconstrictors, anti-migraine drugs, anti-coagulants and anti-thrombotic drugs, anti-pyretics, hypnotics, sedatives, anticonvulsants, anti-epileptics, neuromuscular drugs, drugs acting on Central Nervous System, hyper- and hypoglycemic agents, diuretics, anti-obesity drugs, anabolic drugs, anti-uricemic drugs and combinations thereof.

In a specific embodiment, the anti-cancer drug is a taxoid, preferably selected from paclitaxel, docetaxel, their derivatives, analogs and prodrugs.

When the taxoid is paclitaxel, it is present in a relative proportion between 0.5 and 4% (w/w) of the final composition, preferably between 1.5 and 3% (w/w).

According to one specific embodiment of the invention, preferred pharmaceutical composition for oral use comprises an emulsion including vitamin E, D-α-tocopheryl polyethylene glycol succinate 1000 (TPGS), tyloxapol and at least, one therapeutic agent.

The relative proportions of vitamin E, TPGS and tyloxapol may be respectively 2-6, 0-60 and 5-70 (w/w) of the final composition, preferably respectively 2-6, 5-60 and 5-70(w/w) of the final composition, more preferably respectively 3-5, 20-40 and 20-40%.

When the composition is used for intravenous route it contains no TPGS.

According to another specific embodiment of the invention, the relative proportion of propylene glycol is in the range of 0-50% (w/w) of the final composition, preferably equal to 20% (w/w) and the relative proportion of ethanol is in the range of 5-50% (w/w) of the final composition, preferably equal to 30% (w/w).

According to the instant invention, the enhancer is one well-known from the man skilled in the art. It is advantageously selected from the group comprising cytochrome P450 2C8 inhibitors, cytochrome P450 3A4 inhibitors, multidrug resistance inhibitors, Pgp inhibitors or non specific inhibitors.

In a specific embodiment, the enhancer is selected from cyclosporine A, its analogs and derivatives.

The compositions according to the invention may be associated with any pharmaceutical excipient to form a dosage form, which can be administered to animals or humans via intravascular, oral, intramuscular, cutaneous and subcutaneous routes. Specifically emulsions according to the invention can be given by any of the following routes among others: intra-abdominal, intra-arterial, intra-articular, intra-capsular, intra-cervical, intra-cranial, intra-ductal, intra-dural, intra-lesional, intra-ocular, intra-locular, intra-lumbar, intra-mural, intra-operative, intra-parietal, intra-peritoneal, intra-plural, intra-pulmonary, intra-spinal, intra-thoracic, intra-tracheal, intra-tympanic, intra-uterine, intra-ventricular, intra-venous or transdermal or can be nebulised using suitable aerosol propellants.

Self-emulsifying systems give emulsions upon dilution in aqueous media. The presence of an oily core in emulsion droplets (nonexistent in micelles) enables to dissolve higher quantity of drugs and provides an encapsulation effect of stabilization. The presence of an oily core (droplets instead of micelles) enables to load bigger quantity of drugs (e.g. paclitaxel) within the SNEOF than traditional micelles-forming systems such as Taxol®. Furthermore, at equivalent drug concentration, the emulsion provides a better stability of paclitaxel (degradation and/or precipitation) than micelles. The self-nanoemulsifying oily formulations give nanoemulsions with droplet size smaller than or equal to 10 nanometers. Incorporation of drugs such as paclitaxel or cyclosporin A within the SNEOFs does not significantly alter the emulsion size nor the self-nanoemulsification process. The surface offered to drug release and absorption is huge and represents more than 600 m² for 1 ml of SNEOF.

The compositions according to the invention have been specifically designed to ensure self-nanoemulsification (viscosity, HLB) and to provide the best drug solubilization properties. Moreover, the surfactant choice was conditioned by the ability to release the drug so as to ensure a linear pharmacokinetic even after oral administration.

In the sense of the present invention, when the pharmacokinetic is linear, the dose of therapeutic agent is proportional to the blood plasma level of the therapeutic agent desired.

Thus, two novel polymeric surfactants were used concomitantly with an oily carrier: tyloxapol (oxyethylated tert-octylphenol formaldehyde polymer) and alpha-tocopheryl polyethylene glycol 1000 succinate (TPGS). Their unique combination in SNEOF ensures a linear release of lyophobic drugs and a linear pharmacokinetic even at high doses.

An other object of the invention is the use of TPGS and tyloxapol for preparing pharmaceutical composition in the form of anhydrous self-nanoemulsifying oily formulation having linear pharmacokinetic even at high doses after oral administration.

Another aspect of the invention is a method of treatment of taxoid-responsive diseases wherein an effective amount of a composition according to the invention is administered to a patient in need thereof.

The advantages of paclitaxel SNEOF according to the instant invention over orally given Taxol^{®} are:
- absence of Cremophor EL,
- better taste, less bitterness,
- 2.5 to 5 fold bigger Paclitaxel content,
- 4 to 8 fold less ethanol at equivalent dose and
- 1.25 to 2.5 fold less ingested volume.

Even low paclitaxel doses of 60 or 90 mg/m² with oral Taxol® lead to bad patient tolerability (nausea, vomiting) (C. M. Kruijtzer, H. Boot, J. H. Beijnen, H. L. Lochs, F. X. Parnis, A. S. Planting, J. M. Pelgrims, R. Williams, R. A. Mathot, H. Rosing, M. E. Schot, H. Van Tinteren, and J. H. Schellens. Weekly oral paclitaxel as first-line treatment in patients with advanced gastric cancer. Ann Oncol 14: 197-204 (2003)). A better tolerability after ingestion is assumed based on a significative reduction of the ethanol content and of the bitterness reduction. Furthermore, selected excipients (TPGS, tyloxapol) have a lower rodent oral DL50 than Cremophor EL. The reduced volume and reduced overall toxicity could safely allow to administrate higher paclitaxel dose to patients than oral Taxol® formulation for which the maximal tolerated dose (MTD) was estimated at 360mg/m² (M. M. Malingre, J. M. Terwogt, J. H. Beijnen, H. Rosing, F. J. Koopman, O. van Tellingen, K. Duchin, W. W. Huinink, M. Swart, J. Lieverst, and J. H. Schellens. Phase I and pharmacokinetic study of oral paclitaxel. J Clin Oncol 18: 2468-2475. (2000)). Moreover the dose-AUC non-linearity and pharmacokinetic variabilities of orally given Taxol® constitute the main limitations to an oral clinical practice use and security (M. M. Malingre, J. H. Beijnen, H. Rosing, F. J. Koopman, O. van Tellingen, K. Duchin, W. W. Ten Bokkel Huinink, M. Swart, J. Lieverst, and J. H. Schellens. A phase I and pharmacokinetic study of bi-daily dosing of oral paclitaxel in combination with cyclosporin A. Cancer Chemother Pharmacol 47: 347-54 (2001)).

The self-nanoemulsifying compositions according to the instant invention may be used for the treatment of different diseases like cancers, tumours, Kaposi's sarcoma, malignancies, uncontrolled tissue or cellular proliferation secondary to tissue injury, and any other disease conditions responsive to taxoids such as paclitaxel and docetaxel, and/or prodrugs and derivatives of the foregoing. Among the types of carcinoma which may be treated particularly effectively with oral paclitaxel, docetaxel, other taxoids, and their prodrugs and derivatives, are hepatocellular carcinoma and liver metastases, cancers of the gastrointestinal tract, pancreas, prostate and lung, and Kaposi's sarcoma. Examples of non-cancerous disease conditions which may be effectively treated with these active agents administered orally in accordance with the present invention are uncontrolled tissue or cellular proliferation secondary to tissue injury, polycystic kidney disease, inflammatory diseases (e. g., arthritis) and malaria.

The novel compositions may be administered in any known pharmaceutical oral dosage form. For example, the formulations may be encapsulated in a soft or hard gelatin capsule or may be administered in the form of a liquid oily preparation. Each dosage form may include, apart from the essential components of the composition conventional pharmaceutical excipients, diluents, sweeteners, flavouring agents, colouring agents and any other inert ingredients regularly included in dosage forms intended for oral administration (see e. g., Remington's Pharmaceutical Sciences, 17th Ed., 1985).

Precise amounts of each of the target drugs included in the oral dosage forms will vary depending on the age, weight, disease and condition of the patient.

Although some of the oral formulations of the invention may provide therapeutic blood levels of the taxoid active ingredient when administered alone, an advantageous method of the invention for treating mammalian patients (particularly human patients) suffering from taxoid-responsive disease conditions is to administer the oral formulations containing the taxoid target agent concomitantly with the administration of at least one dose of an oral bioavailability enhancing agent. This bioenhancer can be concomitantly formulated in the SNEOF or administered separately. Another advantageous method of the invention for treating mammalian patients is to administer the oral formulations containing the taxoid target agent concomitantly or separately with another antitumor agent like carboplatinum and the like.

The preferred embodiment of the method of the invention for oral administration to humans of paclitaxel, its derivatives, analogs and prodrugs, and other taxoids comprises the oral administration of an oral absorption or bioavailability enhancing agent to a human patient simultaneously with, or prior to, or both simultaneously with and prior to the oral administration to increase the quantity of absorption of the intact target agent into the bloodstream.

Different advantages of the present invention will be readily appreciated with the following figures, tables and examples.

Figure 1 illustrates the pharmacokinetic profiles of 10 mg/kg intravenously administered paclitaxel with or without 10mg/kg cyclosporin A oral pre-treatment.

Figure 2 illustrates the pharmacokinetic profiles of oral 10 mg/kg paclitaxel formulations according to the invention with or without 10 mg/kg cyclosporin A oral pre-treatment and compared to paclitaxel alone.

Figure 3 illustrates the pharmacokinetic profiles of oral 10, 30 and 60 mg/kg paclitaxel formulations according to the invention without 10 mg/kg cyclosporin A oral pre-treatment in P-gp knock out mice.

Figure 4 illustrates the pharmacokinetic profiles of oral 10, 30 and 60 mg/kg paclitaxel formulations according to the invention with 10 mg/kg cyclosporin A oral pre-treatment in wild type mice.

### EXAMPLE 1: CHARACTERIZATION OF PACLITAXEL EMULSIONS FOLLOWING 1:10 DILUTION OF SNEOFS WITH WATER

### 1. MATERIALS AND METHODS

### 1.1. Materials

Paclitaxel (MW 853) with 99.34% (w/w) purity (HPLC) was purchased from Farmachem (Lugano, Switzerland). Vitamin E and tyloxapol were bought from Sigma (St. Louis, MO, USA). D-α-tocopheryl polyethylene glycol succinate 1000 (TPGS) was a gift from Eastman Chemical (Kingsport, TN, USA). Ethanol was bought from SDS (Peypin, France). All solvents were HPLC grade

### 1.2. Methods

### Preparation of paclitaxel SNEOFs

SNEOFs were firstly prepared by successive addition and mixing of each excipient. When the oily carrier is clear and homogeneous, paclitaxel is added and quickly dissolved under mild agitation. For combined formulations, Cyclosporin A is lastly added and quickly dissolved under mild agitation in the SNEOF. In the same manner, pH-lowered formulations are obtained by addition of 0.01 or 0.02 % anhydrous citric acid in the previously prepared paclitaxel-containing SNEOF. Its dissolution is slow and requires agitation.

Paclitaxel emulsion may be formed by dilution of SNEOFs with distilled water.

### Examples of paclitaxel SNEOFs

The following compositions have been prepared according to the above-disclosed method.

| **Components** | **% (w, w)** |
|---|---|
| Paclitaxel | 1.5 |
| Vitamin E | 5 |
| TPGS | 31.75 |
| Ethanol anhydrous, absolute | 30 |
| Tyloxapol | 31.75 |

| **Components** | **% (w, w)** |
|---|---|
| Paclitaxel | 3 |
| Vitamin E | 5 |
| TPGS | 31 |
| Ethanol anhydrous, absolute | 30 |
| Tyloxapol | 31 |

| **Components** | **% (w, w)** |
|---|---|
| Paclitaxel | 1.5 |
| Vitamin E | 5 |
| Ethanol anhydrous, absolute | 30 |
| Tyloxapol | 63.5 |

### Examples of combined formulations

The following compositions have been prepared according to the above-disclosed method.

| **Components** | **% (w, w)** |
|---|---|
| Paclitaxel | 1.5 |
| Vitamin E | 5 |
| TPGS | 31 |
| Ethanol anhydrous, absolute | 30 |
| Tyloxapol | 31 |
| Cyclosporin A | 1.5 |

| **Components** | **% (w, w)** |
|---|---|
| Paclitaxel | 3 |
| Vitamin E | 5 |
| TPGS | 30.25 |
| Ethanol anhydrous, absolute | 30 |
| Tyloxapol | 30.25 |
| Cyclosporin A | 1.5 |

| **Components** | **% (w, w)** |
|---|---|
| Paclitaxel | 1.5 |
| Vitamin E | 5 |
| Ethanol anhydrous, absolute | 30 |
| Tyloxapol | 62 |
| Cyclosporin A | 1.5 |

### Examples of pH-lowered formulations

The following compositions have been prepared according to the above-disclosed method.

| **Components** | **% (w, w)** |
|---|---|
| Paclitaxel | 1.5 |
| Vitamin E | 5 |
| TPGS | 31.75 |
| Ethanol anhydrous, absolute | 30 |
| Tyloxapol | 31.75 |
| Citric acid | 0.01 |

### Droplet size

Emulsions were formed following 1:10 dilution of paclitaxel SNEOF with distilled water. The droplet size of the resulting emulsions was determined by the PCS method using a Nanosizer (Malvern, UK)

### Stability Study

SNEOFs containing 1.5 and 3% (w/w) paclitaxel were prepared. A combined form of 1.5% paclitaxel plus 1.5% cyclosporin A was also prepared. The chemical stability of paclitaxel in SNEOFs was monitored using an analytical HPLC method (M. Andreeva, P. D. Iedmann, L. Binder, V. W. Armstrong, H. Meden, M. Binder, M. Oellerich. A simple and reliable reversed-phase high-performance liquid chromatographic procedure for determination of paclitaxel (taxol) in human serum. Ther Drug Monit. 19: 327-32 (1997); A. Sharma, W. D. Conway, R. M. Straubinger. Reversed-phase high-performance liquid chromatographic determination of taxol in mouse plasma. J Chromatogr B Biomed Appl. 655: 315-9 (1994)).

### 2. RESULTS

### 2.1. Physicochemical Characterization

### Droplet size

Following 1:10 dilution of paclitaxel SNEOFs (1.5% and 3% w/w) in distilled water, the droplet size of the resulting nanoemulsions was in average equal to 10 ± 4.0 nm with a low Polydispersity index (<0.15).

### 2.2. Stability Study

The preliminary chemical stability studies indicated that paclitaxel in the SNEOFs was stable at 4, 25 and 40°C. The drug content in SNEOFs at 4, 25 and 40 °C did not change over three months.

### EXAMPLE 2: PHARMACOKINETIC STUDY OF ORAL PACLITAXEL EMULSIONS AFTER CYCLOSPORIN A ORAL PRETREATMENT

### 1. MATERIALS AND METHODS

### 1.1. Materials

### 1.1.1. Animals

- Strains:: FVB Wild-type mice
- Source:: Breeding stocks of the animal facility of the
Netherlands Kancer Institute (NKI)
- Age:: 8-14 weeks
- Body weight:: 18-30 gram
- Gender:: Female
- Housing:: Animal Department of the NKI

### 1.1.2. Drug

- **Oral Formulation**:: Cyclosporin A (Sandimmun®)
- Source:: Novartis
- Vehicle:: Cremophor EL: Ethanol (65:35, v/v)
- Concentration:: **50 mg/mL**
- Route:: Oral (p.o.)
- Dose:: **10 mg/kg**

- **Oral Taxol:**: Paclitaxel (Taxol®)
- Source:: Bristol-Myers Squibb
- Vehicle:: Cremophor EL: Ethanol (1:1; v/v)
- Concentration:: **6 mg/mL** and 2mg/mL after 1:3 dilution in water for
injection (WFI)
- Route:: Oral (p.o.)
- Dose:: **10 mg/kg**

- **Oral Formulation 1:**: Paclitaxel SNEOF
- Source:: Novagali SAS
- Vehicle:: Tyloxapol / TPGS / Ethanol / Vitamin E
- Concentration:: **15 mg/mL** for SEOF and 1.5 mg/mL after 1:10
dilution in WFI
- Route:: Oral (p.o)
- Dose:: **10 mg/kg**

### Oral formulation 1 composition according to the invention

| **Components** | % **(w,** w) |
|---|---|
| Paclitaxel | 1.5 |
| Vitamin E | 5 |
| TPGS | 31.75 |
| Ethanol anhydrous, absolute | 30 |
| Tyloxapol | 31.75 |

### Oral Formulation 2 according to the invention: Paclitaxel /

### Cyclosporin A SNEOF

- Source:: Novagali SAS
- Vehicle:: Tyloxapol / TPGS / Ethanol / Vitamin E
- Concentration:: Paclitaxel: **15 mg/mL** for SEOF and 1.5 mg/mL
- after 1:10 dilution in WFI: Cyclosporin A: **15 mg/mL** for SEOF and 1.5
mg/mL after 1:10 dilution
- Route:: Oral (p.o)
- Dose:: **10 mg/kg** paclitaxel and **10 mg/kg** cyclosporin A

| **Components** | **% (w, w)** |
|---|---|
| Paclitaxel | 1.5 |
| Vitamin E | 5 |
| TPGS | 31 |
| Ethanol anhydrous, absolute | 30 |
| Tyloxapol | 31 |
| Cyclosporin A | 1.5 |

### Oral formulation 2 composition

### I.V. Formulation: Paclitaxel

- Source:: Paclitaxel (pure compound) supplied by Novagali
- Vehicle:: Polysorbate 80: Ethanol (1:1, v/v)
- Concentration:: **6 mg/mL**, 1.5 mg/ml after 1:4 dilution in saline
- Route:: intravenous (i.v.)
- Dose:: **10 mg/kg**

### 1.1.3. Preparation of drug solutions for oral administration

Cyclosporin A (Sandimmun®) was diluted 1:25 in water for injection to yield a final concentration of 2 mg/mL. A volume of 5 µL per gram body weight was administered to the animals resulting in a dose of 10 mg/kg of cyclosporin A.

Paclitaxel self-nanoemulsifying oily formulations (SNEOF) n°1 and 2 were diluted 1:10 in WFI to give a microemulsion. A volume of 6.67 µL per gram (body weight) was administered to the animals resulting in a dose of 10 mg/kg of paclitaxel.

Paclitaxel in Cremophor EL: ethanol (Taxol®) was diluted with water for injection to a final concentration of 1.5 mg/ml (1:4 dilution). A volume of 6.67 µL per gram (body weight) was administered resulting in a dose level of 10 mg/kg of paclitaxel.

### 1.1.4. Preparation of drug solutions for intravenous administrations

The stock solution of paclitaxel in Polysorbate 80: ethanol (1:1; v/v) was diluted 1:4 with saline to achieve a final concentration of 1.5 mg/mL. A volume of 6.67 µL per gram (body weight) was administered to the animals, resulting in a dose level of 10 mg/kg of paclitaxel.

### 1.2. Study set up

| Cohort | CsA oral Pre-treatment | Paclitaxel vehicle | Animal number | Blood sampling times |
|---|---|---|---|---|
| 1 | 10 mg/kg | TAXOL 0.6% | 20 | 1, 2, 4 & 8 h |
| 2 | 10 mg/kg | SNEOF 1.5% | 20 | 1, 2, 4 & 8 h |
| 3 | None | SNEOF 1.5% + 1.5% CsA | 20 | 1, 2, 4 & 8 h |
| 4 | None | PS 80: EtOH 0.6% IV | 24 | 5 & 30 min, 1, 2, 4 & 8 h |
| 5 | 10 mg/kg | PS 80: EtOH 0.6% IV | 24 | 5 & 30 min, 1, 2,4 & 8 h |

Each cohort through 1 to 3 consisted of 20 Wild-type mice and cohorts. Cohort 1 received paclitaxel in the conventional formulation of Cremophor EL and ethanol at a dose of 10 mg/kg.

Cohorts 2 received a paclitaxel microemulsion formulation according to the invention at a dose of 10 mg/kg of paclitaxel.

Cohorts 1 and 2 received a pre-treatment of 10mg/kg cyclosporin A, 30 minutes prior to paclitaxel administration.

Cohort 3 received both cyclosporin and paclitaxel in a microemulsion formulation according to the instant invention at dose levels of 10 mg/kg of paclitaxel and 10 mg/kg of cyclosporin A.

Cohorts 4 and 5 are reference groups for the calculation of the bioavailability and consisted of 24 animals per cohort. They received paclitaxel at a dose of 10 mg/kg by intravenous injection in the tail vein following oral cyclosporin A or cyclosporin A vehicle.

Oral drug administrations was done by stainless steel gavage using a glass syringe (250 µl: Hamilton luer tip) or a disposable polypropylene syringe (1 ml). The gavage was inserted via the oesophagus into the stomach. Cyclosporin A was administered orally 18 ± 2 min prior to oral paclitaxel. At times 1, 2, 4 and 8 h after oral paclitaxel administration, animals (n=5 per time point per group) were anaesthetized with metofane and blood was sampled by cardiac puncture.

Intravenous drug administrations were done by injection into the tail vein using a disposable polypropylene syringe (300 µl) provided with fixed 29 g needle. Cyclosporin A was administered 30 ± 5 min prior to intravenous paclitaxel. At times 5, 30 min, 1, 2, 4 and 8 h after intravenous paclitaxel administration, animals (n=4 per time point per group) were anaesthetized with metofane and blood was sampled by cardiac puncture.

### 1.2.1. Sample collection, handling and storage

At the specified times, animals were anaesthetized using metofane. After fixation on their back, with their chest in an upright position blood was collected by cardiac puncture using a 1 mL polypropylene syringe fitted with 25 g needle. Blood was transferred immediately into tubes containing potassium EDTA as anticoagulant and mixed by inversion. Blood samples were centrifuged vial for 5 min at 4000 g. The supernatant plasma fraction was transferred to a clean tube with appropriate label and stored at -20°C until analyses.

### 1.2.2. Number of animals /samples

Based on previous experience, 5 animals per time point was sufficient to accurately determine the AUC of the plasma concentration-time curves after oral dosing, whereas 4 animals per time point sufficed for intravenous dose groups. Plasma samples were obtained from all animals.

### 1.2.3. Analytical method

Analyses of paclitaxel levels in the plasma samples used a validated HPLC-UV methodology (Sparreboom, A., van Tellingen, O., Nooijen, W.J., and Beijnen, J.H. Determination of paclitaxel and metabolites in mouse plasma, tissues, urine and faeces by semi-automated reversed-phase high-performance liquid chromatography. J Chromatogr B Biomed Appl, 664: 383-391, 1995.).

### 1.2.4. Assay performance control

Test samples were analysed for paclitaxel in singular within an analytical batch, consisting of a set of calibration standards and QC samples. Per series of 40 test samples at least 3 QC samples containing paclitaxel at concentrations over the expected range were analysed in duplicate. Results of batch analysed were accepted if:
- the correlation coefficient (r) of the calibration curve is higher than 0.98.
- at least 4 of the 6 QC samples are within ±20% of their respective nominal values; 2 of the 6 QC samples (not both at the same concentration) may be outside the ±20% of their respective nominal values.

### 1.2.5. Data reprocessing

Chromatographic data acquisition and processing were done using a Chromeleon (v.6) chromatography data station. Calibration lines were fitted by weighed least squares regression analysis using the reciprocal of the squared concentration as the weight factor.

Plasma concentration data were reported. Plasma concentrations versus time curves were fitted using the MEDI\WARE software package (version 3.0) and pharmacokinetic parameters: Area under the plasma concentration-time curve (AUC), Maximum plasma level (Cₘₐₓ), Elimination half-life (t1/2) and Biological availability (F) were calculated.

### 2. RESULTS

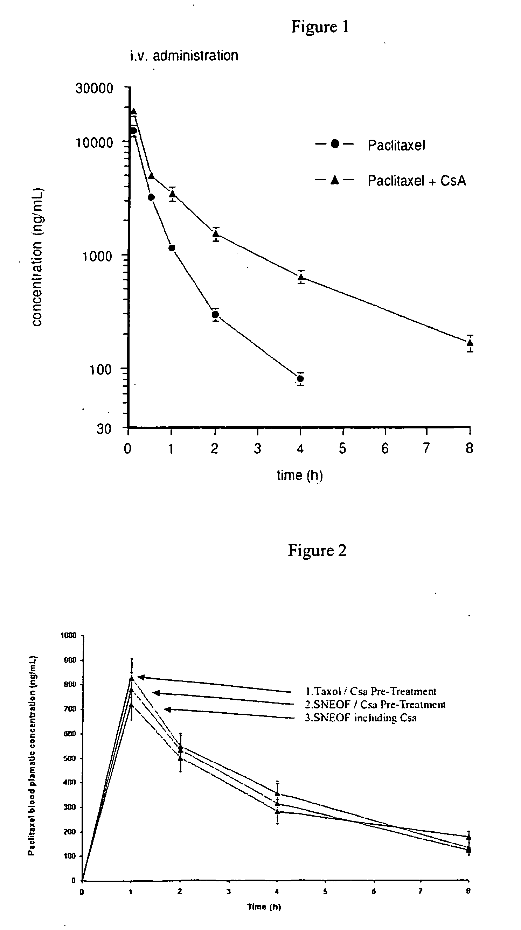

They are illustrated in figures 1 and 2 and in the following table.

Paclitaxel bioavailability of 10mg/kg paclitaxel oral formulations with or without 10 mg/kg oral cyclosporin A pre-medication

| Cohort | CsA oral Pre-treatment | Paclitaxel vehicle | AUC (mean ± SE) (ng/ml.h) | Bioavailability (%) |
|---|---|---|---|---|
| 1 | 10 mg/kg | TAXOL 0.6% | 2984 ± 173 | 21.3 |
| 2 | 10 mg/kg | SNEOF 1.5% | 2670 ± 249 | 19.1 |
| 3 | None | SNEOF 1.5% + 1.5% CsA | 2772 ± 344 | 19.8 |
| 4 | None | PS 80: EtOH 0.6% IV | 5961 ± 374 | 100 |
| 5 | 10 mg/kg | PS 80: EtOH 0.6% IV | 14006 ± 725 | 100 |

### 3. CONCLUSION

Cyclosporin A is known to be an oral taxanes bioenhancer. Oral pre-treatment with cyclosporin A lead to a significant increase of intravenously administered paclitaxel AUC from 5961 ± 374 to 14006 ± 725 (2.35 fold). Oral pre-treatment with cyclosporin A also lead to a significant increase of orally administered paclitaxel AUC (data not shown).

At the dose of 10 mg/kg of paclitaxel plus 10 mg/kg of Cyclosporin A orally administered to wild type mice, both the SNEOF and the Taxol® formulation exhibited an equivalent bioavailability of approximately 20%.

The paclitaxel and cyclosporin A-containing SNEOF also lead to a 20% bioavailability of paclitaxel. This formulation illustrates the possibility to use a combined oral dosage form (SNEOF) of the drug and a bioenhancer.

### EXAMPLE 3: DOSE VERSUS PLASMA - AUC PHARMACOKINETIC STUDY OF ORAL PACLITAXEL EMULSIONS AFTER CYCLOSPORIN A ORAL PRE-TREATMENT IN WILD TYPE MICE OR WITHOUT CYCLOSPORIN ORAL PRE-TREATMENT IN P-GLYCOPROTEIN KNOCK OUT MICE

### 1. MATERIALS AND METHODS

### 1.1. Materials

### 1.1.1. Animals

- Strains:: Mdr1 a/b KO mice and FVB Wild type Mice
- Source:: Breeding stocks of the animal facility of the NKI
- Age:: 8-14 weeks
- Body weight:: 18-30 gram
- Gender:: Female
- Housing:: Animal Department of the NKI

### 1.1.2. Drug

- **Oral Formulation:**: Paclitaxel SNEOF
- Source:: Novagali Pharma SA
- Vehicle:: Tyloxapol / TPGS / Ethanol / Vitamin E
- Concentration:: **15 mg/mL** paclitaxel
- Route:: Oral (p.o)
- Dose:: **10, 30 and 60mg/kg**

| **Components** | **% (w, w)** |
|---|---|
| Paclitaxel | 1.5 |
| Vitamin E | 5 |
| TPGS | 31.75 |
| Ethanol anhydrous, absolute | 30 |
| Tyloxapol | 31.75 |

### Composition of paclitaxel SNEOF

### I.V. Formulation: Paclitaxel

- Source:: Paclitaxel (pure compound) supplied by Novagali
- Vehicle:: Polysorbate 80: Ethanol (1:1, v/v)
- Concentration:: **6 mg/mL,** 1.5 mg/ml after 1:4 dilution in WFI
- Route:: intravenous (i.v.)
- Dose:: **10 mg/kg**

### 1.1.3. Preparation of drug solutions for oral and intravenous administration

Paclitaxel self-nanoemulsifying oily formulations (SNEOF) were diluted 1:10 in WFI to give a microemulsion containing 1.5 mg/ml of paclitaxel. A volume of 6.67 µL per gram (body weight) was administered orally to the animals resulting in a dose of 10 mg/kg of paclitaxel.

Paclitaxel self-nanoemulsifying oily formulations (SNEOF) were diluted 1:10 in WFI to give a microemulsion containing 1.5 mg/ml of paclitaxel. A volume of 20 µL per gram (body weight) was administered orally to the animals resulting in a dose of 30 mg/kg of paclitaxel.

Paclitaxel self-nanoemulsifying oily formulations (SNEOF) were diluted 1:10 in WFI to give a microemulsion containing 1.5 mg/ml of paclitaxel. A volume of 40 µL per gram (body weight) was administered orally to the animals resulting in a dose of 30 mg/kg of paclitaxel.

The stock solution of paclitaxel in Polysorbate 80: ethanol (1:1; v/v) was diluted 1:4 with saline to achieve final concentration of 1.5 mg/mL. A volume of 6.67 µL per gram (body weight) was administered intravenously to the animals, resulting in a dose level of 10 mg/kg of paclitaxel.

### 1.2. Study set up

### 1.2.1. Plasma pharmacokinetic study

Cohorts 12, 13 and 14, received paclitaxel in formulation SNEOF at dose levels of 10, 30 and 60 mg/kg, respectively to investigate the linearity of dose versus plasma AUC relationships of paclitaxel when this drug was given as single agent to P glycoprotein knockout mice, whereas cohorts 15, 16 and 17 received paclitaxel in formulation SNEOF at dose levels of 10, 30 and 60 mg/kg, respectively to investigate the linearity of dose versus plasma AUC relationships of paclitaxel when this drug was given after a Cyclosporin A pre-treatment to Wild type Mice. Each cohort consisted of 20 mice.

### 1.2.2. Sample collection, handling and storage

### Plasma pharmacokinetic study

At the specified times, animals were anaesthetized using metofane. After fixation on their back, with their chest in an upright position blood was collected by cardiac puncture using a 1 mL polypropylene syringe fitted with 25 g needle. Blood was transferred immediately into tubes containing potassium EDTA as anticoagulant and mixed by inversion. Blood samples were centrifuged vial for 5 min at 4000 g. The supernatant plasma fraction was transferred to a clean tube with appropriate label and stored at -20°C until analyses.

5 animals per time point were used to determine the AUC of the plasma concentration-time curves after oral dosing, whereas 4 animals per time point were used for intravenous dose groups. Plasma samples for subsequent analysis were obtained from all animals.

### 1.3. Analytical method

Analyses of paclitaxel levels in the plasma samples were a validated HPLC - UV methodology (Sparreboom, A., van Tellingen, O., Nooijen, W.J., and Beijnen, J.H. Determination of paclitaxel and metabolites in mouse plasma, tissues, urine and faeces by semi-automated reversed-phase high-performance liquid chromatography. J. Chromatogr B. Biomed Appl., 664: 383-391, 1995).

### 1.3.1. Assay performance control

Test samples were analysed for paclitaxel in singular within an analytical batch, consisting of a set of calibration standards and QC samples. Per series of 60 test samples at least 3, QC samples containing paclitaxel at concentrations over the expected range were analysed in duplicate. Results of batch analysed were accepted if:
- the correlation coefficient (r) of the calibration curve is higher than 0.98.
- at least 4 of the 6 QC samples are within ±20% of their respective nominal values; 2 of the 6 QC samples (not both at the same concentration) may be outside the ±20% of their respective nominal values.

### 1.3.2. Data reprocessing

Chromatographic data acquisition and processing were done using a Chromeleon (v.6) chromatography data station. Calibration lines were fitted by weighed least squares regression analysis.

Plasma concentration data were reported. Plasma concentrations versus time curves were fitted using the MEDI\WARE software package (version 3.0) and pharmacokinetic parameters: Area under the plasma concentration-time curve (AUC), Maximum plasma level (Cₘₐₓ), Elimination half-life (t1/2) and Biological availability (F) were calculated.

### 2. RESULTS

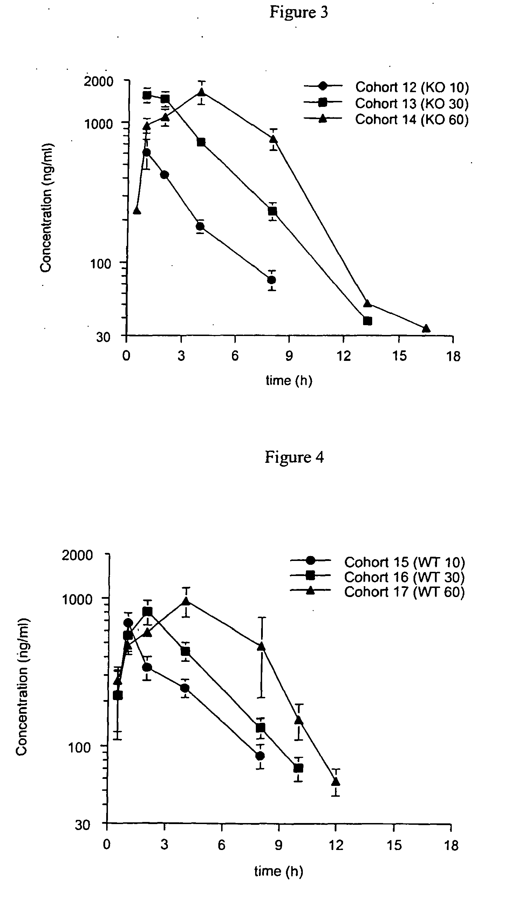

Results are given in the following table and in figure 3 and 4.
Paclitaxel AUC and Bioavailability of oral formulations without Cyclosporine A pre-treatment in Pgp Knock Out Mice and after Cyclosporin Pre-treatment in Wild type mice

| Cohort | CsA | Genotype | Formulation | Route | AUC (mean ± SE) ng/ml* h | Bioavailability % |
|---|---|---|---|---|---|---|
| 12 | No | Mdr1a/b KO | SNEOF (10 mg/kg) | p.o. | 1935 ± 170 | 31.5 ± 3.1 |
| 13 | No | Mdr1a/b KO | SNEOF (30 mg/kg) | p.o. | 7110 ± 382 | 38.6 ± 2.8 |
| 14 | No | Mdr1a/b KO | SNEOF (60 mg/kg) | p.o. | 11229 ± 1130 | 30.4 ± 1.5 |
| 15 | Yes | Wild type | SNEOF (10 mg/kg) | p.o. | 2040 ± 174 | 14.8 ± 1.5 |
| 16 | Yes | Wild type | SNEOF (30 mg/kg) | p.o. | 3522 ± 322 | 8.5 ± 0.9 |
| 17 | Yes | Wild type | SNEOF (60 mg/kg) | p.o. | 5916 ± 765 | 7.2 ± 1.0 |
| 18 | No | Mdrla/b KO | Polysorbate 80: EtOH | i.v. | 6147 ± 291 | |

### 3. CONCLUSION

As it can be observed from the Table the mean oral bioavailability of paclitaxel in SNEOF in P glycoprotein knockout mice ranges from 30.4 to 38.6% and the oral bioavailability appears to be linear over the tested dose range as the differences in mean oral bioavailability are not statistically different.

The AUC after 10 mg/kg of paclitaxel in wild-type mice with cyclosporine A was similar as in knockout mice receiving the same dose. Since the concomitant administration of cyclosporine A also increased AUC of paclitaxel given i.v. (e.g. by inhibition of (metabolic) elimination), the oral bioavailability in wild-type mice was lower. Moreover, the oral bioavailability in wild-type animals was not linear but decreased significantly with dose. In this study, the dose level of cyclosporin A was kept constant at 10 mg/kg and it is possible that the concentration of this competitive P gp inhibitor is insufficient when using higher dose levels of paclitaxel.

The time that the peak plasma level is reached (Tmax) increases at higher dose levels, which suggests that there may be an effect on stomach emptying and/or intestinal transit speed in mice.

## Claims

1. A pharmaceutical composition in a form of an anhydrous self-nanoemulsifying oily formulation comprising:
- one or more therapeutic agent(s) which have low solubility in water or are water-insoluble,
- vitamin E,
- one co-solvent selected from propylene glycol and ethanol and mixture thereof
- one surfactant selected from tyloxapol and from mixture of tyloxapol and TPGS, and optionally,
- a bioenhancer,
provided that when no bioenhancer is present in the composition and said co-solvent is propylene glycol or ethanol, then the composition does not comprise bile salts.

2. A pharmaceutical composition according to claim 1 further comprising an acidic pH adjuster.

3. A pharmaceutical composition according to anyone of claims 1 to 2, wherein vitamin E is from 2 to 6% (w/w) of the final composition.

4. A pharmaceutical composition according to anyone of claims 1 to 3, wherein the one or more therapeutic agent(s) is selected from the group comprising anti-fungal drugs, anti-viral drugs, antibiotic drugs, anti-inflammatory drugs, anti-cancer drugs, analgesics, antidepressants, antipsychotics, hormones, antacids, coronary vasodilators, cerebral vasodilators, psychotropics, antineoplastics, stimulants, anti-histamines, vasodilators, anti-arrythmics, anti-hypertensive drugs, vasoconstrictors, anti-migraine drugs, anti-coagulants and anti-thrombotic drugs, anti-pyretics, hypnotics, sedatives, anticonvulsants, anti-epileptics, neuromuscular drugs, drugs acting on Central Nervous System, hyper- and hypoglycemic agents, diuretics, anti-obesity drugs, anabolic drugs, anti-uricemic drugs, immunosuppressant drugs and combinations thereof.

5. A pharmaceutical composition according to anyone of claims 1 to 4, wherein the one or more therapeutic agent(s) is selected from the group comprising anti-cancer drugs, antineoplastic drugs and combinations thereof.

6. A pharmaceutical composition according to anyone of claims 1 to 5, wherein the anti-cancer drug is a taxoid, preferably selected from paclitaxel, docetaxel, their derivatives, analogs and prodrugs.

7. A pharmaceutical composition according to anyone of claims 1 to 6, wherein the taxoid is paclitaxel in a relative proportion between 0.5 and 4% (w/w) of the final composition, preferably between 1.5 and 3% (w/w).

8. A pharmaceutical composition according to anyone of claims 1 to 7, wherein the relative proportions of vitamin E, TPGS and tyloxapol are respectively 2-6, 0-60 and 5-70 (w/w) of the final composition, preferably respectively 2-6, 5-60 and 5-70 (w/w) of the final composition, more preferably respectively 3-5, 20-40 and 20-40%.

9. A pharmaceutical composition according to anyone of claim 1 to 8 wherein the relative proportion of propylene glycol is in the range of 0-50% (w/w) of the final composition, preferably equal to 20% (w/w) and the relative proportion of ethanol is in the range of 5-50% (w/w) of the final composition, preferably equal to 30% (w/w).

10. A pharmaceutical composition according to anyone of claims 1 to 9, wherein the enhancer is selected from the group comprising cytochrome P450 2C8 inhibitors, cytochrome P450 3A4 inhibitors, multidrug resistance inhibitors, Pgp inhibitors or non specific inhibitors.

11. A pharmaceutical composition according to claim 10, wherein the enhancer is cyclosporine A, its analogs and derivatives.

12. A pharmaceutical composition according to anyone of claims 2 to 11, wherein the acidic pH adjuster is anhydrous citric acid.

13. A pharmaceutical dosage form comprising an anhydrous self-nanoemulsifying oily formulation composition according to anyone of claims 1 to 12 associated to suitable pharmaceutical excipients.

14. A pharmaceutical dosage form according to claim 13, which is suitable for the oral route.

15. A pharmaceutical dosage form according to claim 14 wherein the composition is encapsulated in a soft or hard gelatin capsule or is a liquid oily preparation.

16. A pharmaceutical dosage form according to claim 13, which is suitable for the intravenous route.

17. Use of an anhydrous self-nanoemulsifying oily formulation according to anyone of claims 1 to 12 for the manufacture of a medicament useful in the treatment of taxoid-responsive diseases.

18. Use according to claim 17 for administration to patients receiving simultaneously with, concomitantly or prior to, bioavailability enhancing agent and/or another antitumor agent.

19. Use of an anhydrous self-nanoemulsifying oily formulation according to anyone of claims 1 to 12 for the manufacture of a medicament wherein the dose of the therapeutic agent administered is linearly proportional to the blood plasma level of the therapeutic agent desired.

20. Use of tyloxapol and of mixture of tyloxapol and TPGS, for preparing pharmaceutical composition in the form of anhydrous self-nanoemulsifying oily formulation suitable for preparing a medicament wherein the dose of the therapeutic agent administered is linearly proportional to the blood plasma level of the therapeutic agent desired.

## Patentansprüche

1. Pharmazeutische Zusammensetzung in Form einer wasserfreien selbst-nanoemulgierenden öligen Formulierung umfassend:
- einen oder mehrere Wirkstoffe, die eine geringe Wasserlöslichkeit aufweisen oder wasserunlöslich sind,
- Vitamin E,
- ein Co-Lösungsmittel ausgewählt aus Propylenglycol und Ethanol und einer Mischung davon,
- ein Tensid ausgewählt aus Tyloxapol und aus einer Mischung aus Tyloxapol und TPGS, und gegebenenfalls
- einen Bioverstärker,
mit der Maßgabe, daß, wenn in der Zusammensetzung kein Bioverstärker zugegen ist und das Co-Lösungsmittel Propylenglycol oder Ethanol ist, die Zusammensetzung keine Gallensäuresalze enthält.

2. Pharmazeutische Zusammensetzung gemäß Anspruch 1, weiterhin umfassend einen sauren pH-Justierer.

3. Pharmazeutische Zusammensetzung gemäß einem der Ansprüche 1 bis 2, wobei Vitamin E in 2 bis 6 % (G/G) der Endzusammensetzung enthalten ist.

4. Pharmazeutische Zusammensetzung gemäß einem der Ansprüche 1 bis 3, wobei der oder die Wirkstoffe ausgewählt sind aus der Gruppe bestehend aus antifungalen Wirkstoffen, antiviralen Wirkstoffen, Antibiotika, antiinflammatorischen Wirkstoffen, Antikrebswirkstoffen, Analgetika, Antidepressiva, Antipsychotika, Hormonen, säurebindenden Mitteln, koronaren Vasodilatoren, zerebralen Vasodilatoren, Psychopharmaka, Antineoplastika, Stimulanzien, Antihistaminika, Vasodilatoren, Antiarrythmika, antihypertensiven Wirkstoffen, Vasokonstriktoren, Antimigränewirkstoffen, Antikoagulanzien und antithrombotischen Wirkstoffen, Antipyretika, Hypnotika, Sedativa, Antikonvulsiva, Antiepileptika, neuromuskulären Wirkstoffen, Wirkstoffen, die auf das zentrale Nervensystem einwirken, hyper- und hypoglykämischen Mitteln, Diuretika, Antiadipositaswirkstoffen, anabolischen Wirkstoffen, antiuriazedemischen Wirkstoffen, Immunosuppressiva und Kombinationen davon.

5. Pharmazeutische Zusammensetzung gemäß einem der Ansprüche 1 bis 4, wobei der oder die Wirkstoffe ausgewählt sind aus der Gruppe bestehend aus Antikrebswirkstoffen, antineoplastischen Wirkstoffen und Kombinationen davon.

6. Pharmazeutische Zusammensetzung gemäß einem der Ansprüche 1 bis 5, wobei der Antikrebswirkstoff ein Taxoid, bevorzugt ausgewählt aus Paclitaxel, Docetaxel, deren Derivaten, Analoga und Prodrugs ist.

7. Pharmazeutische Zusammensetzung gemäß einem der Ansprüche 1 bis 6, wobei das Taxoid Paclitaxel ist und in einem relativen Anteil zwischen 0,5 und 4 % (G/G) der Endzusammensetzung, bevorzugt zwischen 1,5 und 3 % (G/G) enthalten ist.

8. Pharmazeutische Zusammensetzung gemäß einem der Ansprüche 1 bis 7, wobei die relativen Anteile von Vitamin E, TPGS und Tyloxapol 2 bis 6, 0 bis 60 bzw. 5 bis 70 (G/G), bevorzugt 2 bis 6, 5 bis 60 bzw. 5 bis 70 (G/G) und mehr bevorzugt 3 bis 5, 20 bis 40 bzw. 20 bis 40 % der Endzusammensetzung beträgt.

9. Pharmazeutische Zusammensetzung gemäß einem der Ansprüche 1 bis 8, wobei der relative Anteil von Propylenglycol im Bereich von 0 bis 50 % (G/G), bevorzugt gleich 20 % (G/G) der Endzusammensetzung beträgt, und der relative Anteil von Ethanol im Bereich von 5 bis 50 % (G/G), bevorzugt gleich 30 % (G/G) der Endzusammensetzung beträgt.

10. Pharmazeutische Zusammensetzung gemäß einem der Ansprüche 1 bis 9, wobei der Verstärker ausgewählt ist aus der Gruppe bestehend aus Cytochrom P450 2C8-Inhibitoren, Cytochrom P450 3A4-Inhibitoren, Multiwirkstoff-Resistenz-Inhibitoren, Pgp-Inhibitoren oder nichtspezifischen Inhibitoren.

11. Pharmazeutische Zusammensetzung gemäß Anspruch 10, wobei der Verstärker Cyclosporin A, Analoga davon und Derivate davon ist.

12. Pharmazeutische Zusammensetzung gemäß einem der Ansprüche 2 bis 11, wobei der saure pH-Justierer wasserfreie Zitronensäure ist.

13. Pharmazeutische Darreichungsform, umfassend eine wasserfreie selbst-nanoemulgierende ölige Formulierungszusammensetzung gemäß einem der Ansprüche 1 bis 12, zusammen mit geeigneten pharmazeutischen Trägerstoffen.

14. Pharmazeutische Darreichungsform gemäß Anspruch 13, die für die orale Route geeignet ist.

15. Pharmazeutische Darreichungsform gemäß Anspruch 14, wobei die Zusammensetzung in einer weichen oder harten Gelatinekapsel verkapselt oder eine flüssige ölige Zubereitung ist.

16. Pharmazeutische Darreichungsform gemäß Anspruch 13, die für die intravenöse Route geeignet ist.

17. Verwendung einer wasserfreien selbst-nanoemulgierenden öligen Formulierung gemäß einem der Ansprüche 1 bis 12 zur Herstellung eines Medikaments zur Verwendung in der Behandlung von auf Taxoid reagierenden Krankheiten.

18. Verwendung gemäß Anspruch 17 zur Verabreichung an Patienten, die gleichzeitig dazu, begleitend oder davor Mittel zur Erhöhung der Bioverfügbarkeit und/oder andere Antitumormittel erhalten.

19. Verwendung einer wasserfreien selbst-nanoemulgierenden öligen Formulierung gemäß einem der Ansprüche 1 bis 12 zur Herstellung eines Medikaments, wobei die Dosis des verabreichten Wirkstoffs linear proportional zum Blutplasmaspiegel des gewünschten Wirkstoffs ist.

20. Verwendung von Tyloxapol und Mischungen von Tyloxapol und TPGS zur Herstellung einer pharmazeutischen Zusammensetzung in Form einer wasserfreien selbst-nanoemulgierenden öligen Formulierung, die zur Herstellung eines Medikaments geeignet ist, wobei die Dosis des verabreichten Wirkstoffs linear proportional zum Blutplasmaspiegel des gewünschten Wirkstoffs ist.

## Revendications

1. Composition pharmaceutique sous la forme d'une forme galénique huileuse auto-nanoémulsifiante anhydre comprenant :
- un ou plusieurs agents thérapeutiques, qui ont une faible solubilité dans l'eau ou sont insolubles dans l'eau,
- de la vitamine E,
- un co-solvant choisi parmi le propylène glycol et l'éthanol et leur mélange,
- un tensioactif choisi parmi le tylopaxol et un mélange de tylopaxol et de TPGS, et éventuellement,
- un bio-amplificateur,
pourvu que quand aucun bio-amplificateur n'est présent dans la composition et que ledit co-solvant est le propylène glycol ou l'éthanol, alors la composition ne comprend pas de sels biliaires.

2. Composition pharmaceutique selon la revendication 1 comprenant en outre un ajusteur de pH acide.

3. Composition pharmaceutique selon l'une quelconque des revendications 1 à 2, dans laquelle la vitamine E constitue de 2 à 6 % (p/p) de la composition finale.

4. Composition pharmaceutique selon l'une quelconque des revendications 1 à 3, dans laquelle le ou les agent(s) thérapeutique(s) est choisi dans le groupe comprenant les médicaments anti-fongiques, les médicaments anti-viraux, les médicaments antibiotiques, les médicaments anti-inflammatoires, les médicaments anti-cancéreux, les analgésiques, les antidépresseurs, les antipsychotiques, les hormones, les antiacides, les vasodilatateurs coronaires, les vasodilatateurs cérébraux, les psychotropes, les antinéoplasiques, les stimulants, les anti-histaminiques, les vasodilatateurs, les anti-arythmiques, les médicaments anti-hypertenseurs, les vasoconstricteurs, les médicaments anti-migraine, les anticoagulants et les médicaments anti-thrombotiques, les anti-pyrétiques, les hypnotiques, les sédatifs, les anticonvulsivants, les anti-épileptiques, les médicaments neuro-musculaires, les médicaments agissant sur le système nerveux central, les agents hyper- et hypoglycémiques, les diurétiques, les médicaments anti-obésité, les médicaments anaboliques, les médicaments anti-uricémiques, les médicaments immunosuppresseurs et leurs combinaisons.

5. Composition pharmaceutique selon l'une quelconque des revendications 1 à 4, dans laquelle le un ou plusieurs agents thérapeutiques est choisi dans le groupe comprenant les médicaments anti-cancéreux, les médicaments anti-néoplasiques et leurs combinaisons.

6. Composition pharmaceutique selon l'une quelconque des revendications 1 à 5, dans laquelle le médicament anti-cancéreux est un taxoïde, choisi de préférence parmi le paclitaxel, le docetaxel, leurs dérivés, analogues et pro-drogues.

7. Composition pharmaceutique selon l'une quelconque des revendications 1 à 6, dans laquelle le taxoïde est le paclitaxel en proportion relative entre 0,5 et 4 % (p/p) de la composition finale, de préférence entre 1,5 et 3 % (p/p).

8. Composition pharmaceutique selon l'une quelconque des revendications 1 à 7, dans laquelle les proportions relatives de vitamine E, TPGS et tyloxapol sont respectivement de 2 à 6, 0 à 60 et 5 à 70 (p/p) de la composition finale, de préférence respectivement de 2 à 6, 5 à 60 et 5 à 70 (p/p) de la composition finale, davantage de préférence respectivement de 3 à 5, 20 à 40 et 20 à 40 %.

9. Composition pharmaceutique selon l'une quelconque des revendications 1 à 8, dans laquelle la proportion relative de propylène glycol se situe dans la plage allant de 0 à 50 % (p/p) de la composition finale, de préférence égale à 20 % (p/p) et la proportion relative d'éthanol se situe dans la plage allant de 5 à 50 % (p/p) de la composition finale, de préférence égale à 30 % (p/p).

10. Composition pharmaceutique selon l'une quelconque des revendications 1 à 9, dans laquelle l'amplificateur est choisi dans le groupe comprenant les inhibiteurs du cytochrome P450 2C8, les inhibiteurs du cytochrome P450 3A4, les inhibiteurs de la résistance à des médicaments multiples, les inhibiteurs du Pgp ou des inhibiteurs non spécifiques.

11. Composition pharmaceutique selon l'une quelconque des revendications 1 à 10, dans laquelle l'amplificateur est la cyclosporine A, ses analogues et dérivés.

12. Composition pharmaceutique selon l'une quelconque des revendications 2 à 11, dans laquelle l'ajusteur de pH acide est l'acide citrique anhydre.

13. Forme pharmaceutique comprenant une composition de forme galénique huileuse auto-nanoémulsifiante anhydre selon l'une quelconque des revendications 1 à 12 associée à des excipients pharmaceutiques adaptés.

14. Forme pharmaceutique selon la revendication 13, qui est adaptée pour la voie orale.

15. Forme pharmaceutique selon la revendication 14, dans laquelle la composition est encapsulée dans une capsule de gélatine molle ou dure ou est une préparation huileuse liquide.

16. Forme pharmaceutique selon la revendication 13, qui est adaptée pour la voie intraveineuse.

17. Utilisation d'une forme galénique huileuse auto-nanoémulsifiante anhydre selon l'une quelconque des revendications 1 à 12 pour la fabrication d'un médicament utile dans le traitement des maladies sensibles aux taxoïdes.

18. Utilisation selon la revendication 17 pour l'administration à des patients recevant simultanément avec, concomitamment à ou avant l'agent amplificateur de biodisponibilité et/ou un autre agent tumoral.

19. Utilisation d'une forme galénique huileuse auto-nanoémulsifiante anhydre selon l'une quelconque des revendications 1 à 12 pour la fabrication d'un médicament dans lequel la dose de l'agent thérapeutique administrée est linéairement proportionnelle au niveau de plasma sanguin de l'agent thérapeutique souhaité.

20. Utilisation du tyloxapol et du mélange de tyloxapol et de TPGS pour préparer une composition pharmaceutique sous la forme d'une forme galénique huileuse auto-nanoémulsifiante anhydre adaptée pour préparer un médicament dans lequel la dose de l'agent thérapeutique administré est linéairement proportionnelle au niveau de plasma sanguin de l'agent thérapeutique souhaité.
